Europäisches Patentamt

European Patent Office

Office européen des brevets

(19)

(11) **EP 0 911 044 B1**

(12) **EUROPÄISCHE PATENTSCHRIFT**

(45) Veröffentlichungstag und Bekanntmachung des Hinweises auf die Patenterteilung:
**22.12.2004 Patentblatt 2004/52**

(51) Int Cl.$^7$: **A61M 1/36**, A61B 5/0285

(21) Anmeldenummer: **98119568.8**

(22) Anmeldetag: **16.10.1998**

(54) **Verfahren und Vorrichtung zur kontinuierlichen Überwachung einer extrakorporalen Blutbehandlung**

Means and method for continuously monitoring an extracorporeal blood treatment

Procédé et dispositif pour contrôler d'une façon continue un traitement extracorporel de sang

(84) Benannte Vertragsstaaten:
**DE ES FR GB IT**

(30) Priorität: **21.10.1997 DE 19746377**

(43) Veröffentlichungstag der Anmeldung:
**28.04.1999 Patentblatt 1999/17**

(73) Patentinhaber: **Fresenius Medical Care Deutschland GmbH**
**61352 Bad Homburg v.d.H. (DE)**

(72) Erfinder: **Spickermann, Reiner**
**97535 Wasserlosen/Burghausen (DE)**

(74) Vertreter: **Luderschmidt, Schüler & Partner GbR Patentanwälte,**
**John-F.-Kennedy-Strasse 4**
**65189 Wiesbaden (DE)**

(56) Entgegenhaltungen:
**US-A- 4 710 164**       **US-A- 5 564 427**

## Beschreibung

Die Erfindung betrifft eine Vorrichtung zur extrakorporalen Blutbehandlung mit einer Einrichtung zur kontinuierlichen Überwachung einer extrakorporalen Blutbehandlung, insbesondere eine Blutbehandlungsvorrichtung, bei der Blut in einem extrakorporalen Kreislauf eine Blutkammer eines durch eine semipermeable Membran in die Blutkammer und eine Dialysierflüssigkeitskammer bzw. Hämofiltratkammer unterteilten Dialysators bzw. Hämofilters durchströmt.

Akute Notfälle während der Dialyse verlangen nach sofortigem Handeln. Sie können blutbehandlungsunabhängig aber auch bedingt durch die Blutbehandlung selbst auftreten. Eine Hauptkomplikation während der Dialysebehandlung und/oder der Hämofiltration stellt der Abfall des Blutdrucks dar. Die häufigste Ursache für einen derartigen Zwischenfall ist eine Hypovolämie in Folge eines zu intensiven Flüssigkeitsentzuges. Andere Ursachen sind in Konzentrationsveränderungen, Temperaturveränderungen oder auch in Bioinkompatibilitätsreaktio- nen zu suchen (Journal für das Nephrologische Team 3-1996-5. 113).

Es sind Hämodialysegeräte bekannt, die über einen Einschub verfügen, der die indirekte Messung des systolischen und diastolischen Blutdrucks des Patienten erlaubt. Es handelt sich dabei um ein konventionelles Blutdruckmeßgerät mit einer aufblasbaren Gummimanschette, die mit einem Manometer verbunden ist. Zur Messung des systolischen Blutdrucks wird der Manschettendruck langsam erhöht, bis der Puls nicht mehr zu tasten ist. Wird der Manschettendruck daraufhin langsam erniedrigt, bis der erste Pulsschlag tastbar ist, kann der systolische Druck gemessen werden. Fällt der Manschettendruck gerade unter den systolischen Blutdruck, so treten pulssynchrone Gefäßgeräusche auf, die bei weiterem Ablassen des Manschettendrucks nicht mehr hörbar sind, wenn der diastolische Druck erreicht ist. Die Messung erfolgt vollautomatisch mit einem akustischen Sensor, einem Drucksensor und einer elektrischen Luftpumpe zum Aufblasen der Manschette. Nachteilig ist, daß der Blutdruck bei den bekannten Dialysegeräten mit dem konventionellen Blutdruckmeßgerät nur in bestimmten Zeitabständen überwacht wird. Zwischen zwei Messungen liegt im allgemeinen ein Zeitraum von ca. 10 bis 15 Minuten. Kürzere Zeitabstände im Minutenbereich sind zwar möglich, doch wäre dies für den Patienten während einer Hämodialysebehandlung mit einer üblichen Dauer von mehreren Stunden nicht zumutbar. Innerhalb dieses Zeitraums können Anzeichen eines Kreislaufproblems unerkannt bleiben, die darauffolgende Messung dagegen zur Einleitung einer entsprechenden Gegenreaktion zu spät sein, um die Behandlung ohne Unterbrechung fortführen zu können.

Zur kontinuierlichen, nichtinvasiven Überwachung von Änderungen des Blutdrucks ist ein Verfahren bekannt, das auf der Analyse der Pulswellenlaufzeit beruht (Psychophysiology, 1976, Vol. 13, No. 1). Bei dem bekannten Verfahren wird von der Laufzeit, die eine durch Herzkontraktion erzeugte Welle braucht, bis sie eine bestimmte Stelle des Körpers erreicht, auf die Höhe des Blutdrucks geschlossen. Verschiedene Untersuchungen bestätigen, daß zwischen der Pulswellenlaufzeit und dem systolischen und diastolischen bzw. mittleren Blutdruck ein annähernd linearer Zusammenhang besteht.

Aus der US-A-4,710,164 ist eine Dialysevorrichtung bekannt, die über eine Einrichtung zum Messen des Blutdrucks verfügt. Bei Unterschreiten eines voreingestellten Grenzwertes wird ein Eingriff in den Behandlungsablauf vorgenommen, der beispielsweise in der Reduzierung der Ultrafiltrationsrate liegen kann.

Die US-A-5,564,427 beschreibt eine Einrichtung zur nichtinvasiven Messung des Blutdrucks, bei der die Ausbreitgeschwindigkeit bzw. Laufzeit der Pulswellen erfasst wird. Es wird vorgeschlagen, die Pulswellen mittels einer um den Arm des Patienten zu legenden Manschette zu detektieren. Dies erfordert aber zusätzliche Handgriffe.

Der Erfindung liegt die Aufgabe zugrunde, eine Blutbehandlungsvorrichtung zu schaffen, bei der die Gefahr des Auftretens von Komplikationen aufgrund eines Blutdruckabfalls während der Blutdruckbehandlung verringert ist, ohne dass zur Messung des Blutdrucks besondere Handgriffe, beispielsweise das Anlegen eines Blutdruckmessers erforderlich sind.

Die Lösung dieser Aufgabe erfolgt erfindungsgemäß mit den im Patentanspruch 1 angegebenen Merkmalen.

Bei der erfindungsgemäßen Vorrichtung wird der Blutdruck oder eine mit dem Blutdruck des Patienten korrelierende Größe während der Blutbehandlung kontinuierlich überwacht. Fällt der gemessene Wert stark ab, wird ein Steuersignal zur Einleitung eines Eingriffs in den Behandlungsablauf erzeugt, um dem Blutdruckabfall entgegenwirken zu können. Da die Messung kontinuierlich erfolgt, kann ein Blutdruckabfall sofort erkannt und diesem sofort entgegengewirkt werden.

Die kontinuierliche, nichtinvasive Messung des Blutdrucks beruht auf der Erfassung der Ausbreitungsgeschwindigkeit der sich über das arterielle Gefäßsystem des Patienten fortpflanzenden Pulswellen, die durch dessen Herzkontraktionen erzeugt werden. Anstelle der Ausbreitungsgeschwindigkeit kann auch die Laufzeit der Pulswellen über eine Strecke des Gefäßsystems mit einer vorgegebenen Länge bestimmt werden.

Von Vorteil ist, daß die Überwachung der Blutbehandlung vom Patienten nicht bemerkt wird, zumal eine umständliche Messung des Blutdrucks in bestimmten Zeitabständen mittels einer aufblasbaren Gummimanschette nicht erforderlich ist.

Unter Blutbehandlungseinrichtungen werden alle Einrichtungen verstanden, in denen Blut eines Patienten oder Spenders in einem extrakorporalen Kreislauf einer bestimmten Behandlung unterzogen wird. Zu den Blutbehandlungseinrichtungen zählen nicht nur Einrichtungen zur Hämodialyse oder -filtration, sondern auch die bekannten Zellseperatoren, in denen das Blut eines Spenders einer Zentrifugation unterworfen und in seine Bestandteile getrennt wird.

Zur kontinuierlichen Messung des Blutdrucks wird die Ausbreitungsgeschwindigkeit der Pulswellen oder die Pulswellenlaufzeit bestimmt. Die Mittel zum Bestimmen der Ausbreitungsgeschwindigkeit oder der Pulswellenlaufzeit umfassen eine Einrichtung zur Ermittlung eines Elektrokardiogramms sowie vorteilhafterweise eine Einrichtung zum Bestimmen der Zeit zwischen dem Zeitpunkt, zu dem die sogenannte R-Zacke (maximale Kammerdepolarisation) im Elektrokardiogramm auftritt, und dem Zeitpunkt, zu dem die Pulswelle detektiert wird, die auf die Herzkontraktion zurückzuführen ist. Als Einrichtung zur Detektion der Pulswellen ist der ohnehin in den bekannten Blutbehandlungsvorrichtungen vorhandene arterielle Drucksensor des extrakorporalen Blutkreislaufes vorgesehen.

Bei der erfindungsgemäßen Vorrichtung wird von der Pulswellenlaufzeit auf den Blutdruck geschlossen. Die Mittel zum Bestimmen des Blutdrucks oder einer mit dem Blutdruck korrelierenden Größe umfassen eine Recheneinheit, in der aus der Pulswellenlaufzeit der Blutdruck auf der Grundlage eines linearen Zusammenhangs berechnet wird. Für die relative Überwachung des Blutdrucks ist es ausreichend, eine mit dem Blutdruck korrelierende Größe, wie z.B. die Pulswellenlaufzeit, zu bestimmen. Wenn jedoch absolute Blutdruckwerte gemessen werden sollen, kann eine Kalibrierung mit einem bekannten Blutdruckmessgerät vorgenommen werden.

Für den Fall, dass der gemessene Blutdruck unter einen vorgegebenen Grenzwert abfällt oder eine zu große Änderung des relativen Blutdrucks erfolgt, wird ein Aktivierungs- bzw. Deaktivierungssignal erzeugt, um einen Eingriff in den Behandlungsablauf einzuleiten. Hierzu verfügt die Blutbehandlungsvorrichtung über eine Steuereinheit.

Bei einer bevorzugten Ausführungsform der Blutbehandlungsvorrichtung ist eine Einrichtung zur Applikation eines Bolus, insbesondere einer vorbestimmten Menge einer NaCl-Lösung, in den extrakorporalen Blutkreislauf vorgesehen, die von der Steuereinheit dann aktiviert wird, wenn das Aktivierungs- bzw. Deaktivierungssignal zur Einleitung eines Eingriffs in den Behandlungsablauf erzeugt wird.

Bei einer weiteren bevorzugten Ausführungsform der Blutbehandlungsvorrichtung ist ein akustischer und/oder optischer Alarmgeber vorgesehen, der von der Steuereinheit dann aktiviert wird, wenn das Aktivierungs- bzw. Deaktivierungssignal erzeugt wird.

Bei einer Ausführungsform einer Blutbehandlungsvorrichtung, die über eine Bilanziereinrichtung und eine Ultrafiltrationseinrichtung verfügt, wird die Ultrafiltrationspumpe der Ultrafiltrationseinrichtung von der Steuereinheit dann deaktiviert, wenn der Blutdruck unter den vorgegebenen Grenzwert abfällt. Die Deaktivierung der Ultrafiltration kann für einen vorbestimmten Zeitraum erfolgen, bis der Blutdruck wieder auf einen akzeptablen Wert angestiegen ist.

Bei einer Blutbehandlungsvorrichtung, deren Dialysierflüssigkeitsquelle eine Temperiereinheit aufweist, mit der mindestens zwei unterschiedliche Temperaturwerte vorgegeben werden können, wird die Temperiereinheit von der Steuereinheit vorteilhafterweise auf eine niedrigere Temperatur eingestellt, wenn der Blutdruck unter den vorgegebenen Grenzwert abfällt.

Um dem Blutdruckabfall entgegenzuwirken, kann auch die Elektrolytzusammensetzung, insbesondere $Na^+$ oder $K^+$, verändert, z.B. die Na-Konzentration erhöht werden. Auch ist eine automatische Gabe von blutdruckstabilisierenden Mitteln möglich. Diese Eingriffe in den Behandlungsablauf können von der Blutbehandlungsvorrichtung selbständig vorgenommen werden.

Im folgenden werden zwei Ausführungsbeispiele einer extrakorporalen Blutbehandlungsvorrichtung unter Bezugnahme auf die Zeichnungen näher erläutert.

Es zeigen:

Figur 1
Eine Hämodialysevorrichtung mit einer Einrichtung zur nichtinvasiven, kontinuierlichen Überwachung des Blutdrucks in schematischer Darstellung,

Figuren 2a und 2b
ein Elektrokardiogramm und ein am Finger des Patienten gemessenes Pulswellensignal zur Bestimmung der Pulswellenlaufzeit und

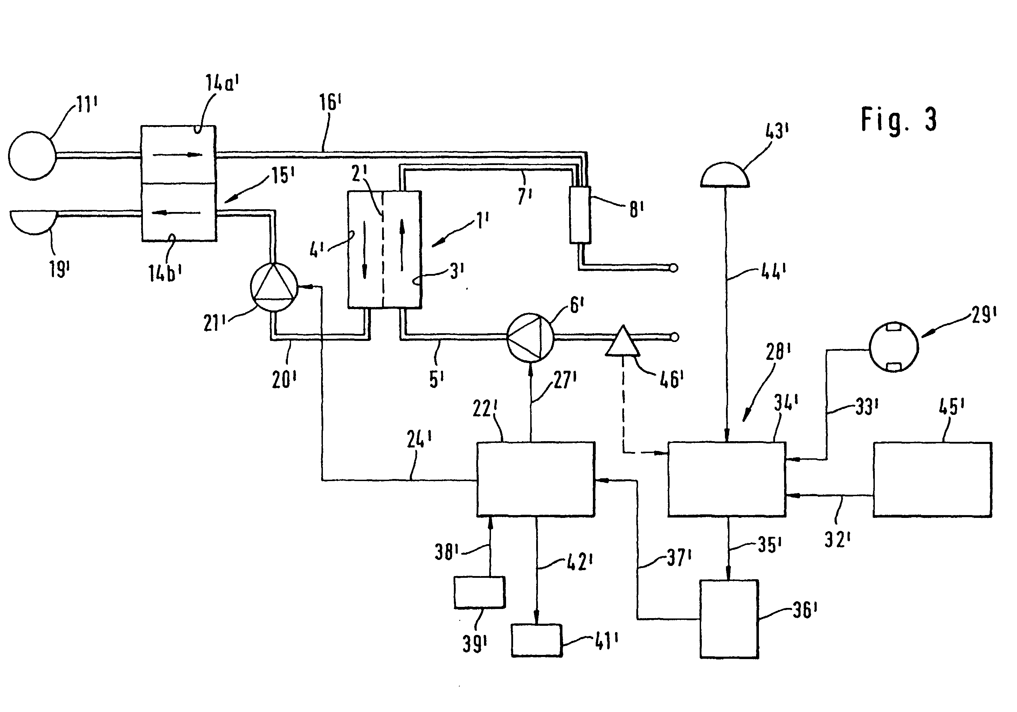

Figur 3 eine Hämofiltrationsvorrichtung mit einer Einrichtung zur nichtinvasiven, kontinuierlichen Überwachung des Blutdrucks in schematischer Darstellung.

Die Hämodialysevorrichtung weist einen Dialysator 1 auf, der durch eine semipermeable Membran 2 in eine Blutkammer 3 und eine Dialysierflüssigkeitskammer 4 getrennt ist. Der Einlaß der Blutkammer ist mit einem Ende einer Blutzuführleitung 5 verbunden, in die eine Blutpumpe 6 geschaltet ist, während der Auslaß der Blutkammer 3 mit dem einen Ende einer Blutabführleitung 7 verbunden ist, in die eine Tropfkammer 8 geschaltet ist. Der extrakorporale Blutkreislauf weist ferner eine Einrichtung 9 zur automatischen Applikation eines Bolus, insbesondere einer physiologischen NaCl-Lösung (typischerweise 200 ml) oder auch online-filtrierter Substituatlösung mit einer Substitutionsrate von typischerweise 150 ml/min auf. Der Bolus wird über eine Zuführleitung 10, die stromauf der Tropfkammer 8 an die Blutzuführleitung 7 angeschlossen ist, dem Patienten zugeführt.

Das Dialysierflüssigkeitssystem der Hämodialysevorrichtung umfaßt eine Einrichtung 11 zur Aufbereitung der Dialysierflüssigkeit, wobei unterschiedliche Zusammensetzungen der Dialysierflüssigkeit (Elektrolytgabe) vorgegeben werden können. Die Dialysierflüssigkeitsaufbereitungseinrichtung 11 verfügt über eine Temperiereinheit 12, mit der die Temperatur der Dialsierflüssigkeit auf verschiedene Werte eingestellt und konstant gehalten werden kann. Sie ist über den ersten Abschnitt 13 einer Dialysierflüssigkeitszuführleitung mit dem Einlaß der ersten Kammerhälfte 14a einer Bilanziereinrichtung 15 verbunden. Der zweite Abschnitt 16 der Dialysierflüssigkeitszuführleitung verbindet den Auslaß der ersten Bilanzierkammerhälfte 14a mit dem Einlaß der Dialysierflüssigkeitskammer 4. Der Auslaß der Dialysierflüssigkeitskammer 4 ist über den ersten Abschnitt 17 einer Dialysierflüssigkeitsabführleitung mit dem Einlaß der zweiten Bilanzierkammerhälfte 14b verbunden. In den ersten Abschnitt 17a der Dialysierflüssigkeitsabführleitung ist eine Dialysierflüssigkeitspumpe 18 geschaltet. Der Auslaß der zweiten Bilanzierkammerhälfte 14b ist über den zweiten Abschnitt 17b der Dialysierflüssigkeitsabführleitung mit einem Auslauf 19 verbunden. Stromauf der Dialysierflüssigkeitspumpe 18 zweigt von der Dialysierflüssigkeitsabführleitung 17 eine Ultrafiltratleitung 20 ab, die ebenfalls zu dem Auslauf 19 führt. In die Ultrafiltratleitung 20 ist eine Ultrafiltrationspumpe 21 geschaltet.

Die Hämodialysevorrichtung umfaßt ferner eine zentrale Steuereinheit 22, die über Steuerleitungen 23 bis 27 mit der Blutpumpe 6, der Dialysierflüssigkeitspumpe 18, der Ultrafiltrationspumpe 21, der Einrichtung 11 zur Aufbereitung der Dialysierflüssigkeit und der Einrichtung 9 zur automatischen Applikation eines Bolus verbunden ist.

Während der Hämodialysebehandlung wird die Blutkammer 3 von dem Blut des Patienten und die Dialysierflüssigkeitskammer 4 des Dialysators 1 von der Dialysierflüssigkeit durchströmt. Da die Bilanziereinrichtung 15 in den Dialysierflüssigkeitsweg geschaltet ist, kann nur soviel Dialysierflüssigkeit über die Dialysierflüssigkeitszuführleitung 16 zufließen, wie Dialysierflüssigkeit über die Dialysierflüssigkeitsabführleitung 17 abfließen kann. Mit der Ultrafiltrationspumpe 21 kann dem Patienten Flüssigkeit entzogen werden.

Die Hämodialysevorrichtung weist darüber hinaus eine Einrichtung 28 zur kontinuierlichen, nichtinvasiven Überwachung der extrakorporalen Blutbehandlung auf. Die Überwachungseinrichtung 28 überwacht den Blutdruck des Patienten während der Dialysebehandlung und erzeugt ein Aktivierungs- bzw. Deaktivierungssignal für die Einleitung eines Eingriffs in den Behandlungsablauf, wenn der Blutdruck des Patienten plötzlich abfällt, um dem Blutdruckabfall entgegenzuwirken.

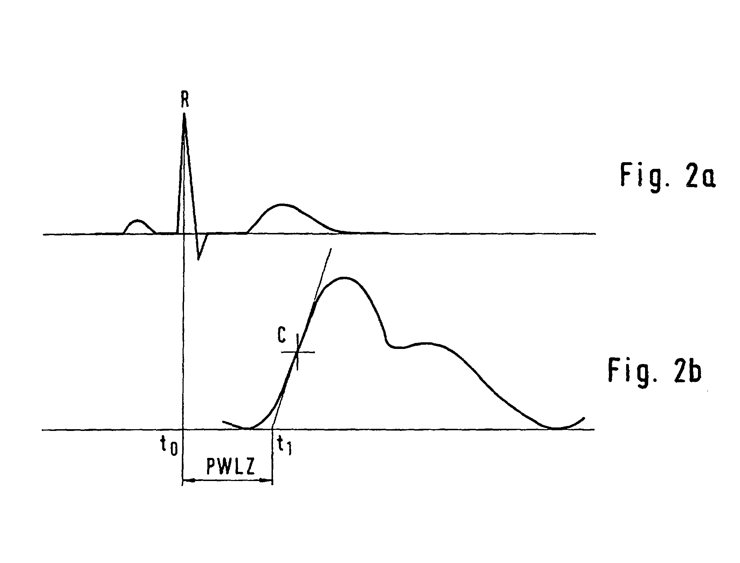

Nachfolgend wird das Meßverfahren zur kontinuierlichen, nichtinvasiven Überwachung des Blutdrucks unter Bezugnahme auf Figur 2 im einzelnen beschrieben.

Die kontinuierliche, nichtinvasive Messung des Blutdrucks beruht auf der Analyse der Pulswellengeschwindigkeit bzw. Pulswellenlaufzeit. Die Blutdruckwelle, die auch als Puls bezeichnet wird, ist auf den Druckanstieg in der Aorta während der Systole zurückzuführen. Sie pflanzt sich über das gesamte arterielle Gefäßsystem mit einer durchschnittlichen Geschwindigkeit von 4 bis 6 m/s fort. Als Pulswellengeschwindigkeit (PWG) wird die Ausbreitungsgeschwindigkeit der Pulswelle im arteriellen Gefäßsystem bezeichnet. Sie nimmt bei erhöhtem Blutdruck zu, da sich die Dehnbarkeit der Gefäßwand infolge des erhöhten Drucks reduziert. Die Pulswellengeschwindigkeit ist der Quotient aus der Länge einer bestimmten Strecke und der Laufzeit der Pulswelle, um diese Strecke zurückzulegen. Zwischen der Pulswellengeschwindigkeit (PWG) und dem Blutdruck besteht der folgende Zusammenhang (Med. & Biol. Eng. & Comput. 1986, 24, 248-254):

$$PWG = \sqrt{\frac{1}{\rho} \cdot \frac{dP}{dV} \cdot V} \qquad (1)$$

wobei V das Blutvolumen ist, dV die Änderung des Blutvolumens, dP die Änderung des Blutdrucks und $\rho$ die Massendichte des Blutes ist.

Zwischen der Pulswellengeschwindigkeit (PWG) bzw. Pulswellenlaufzeit (PWLZ) und dem systolischen und diastolischen oder mittleren Blutdruck (P) besteht ein annähernd linearer Zusammenhang. Der Blutdruck kann aus der Pulswellengeschwindigkeit bzw. Pulswellenlaufzeit annähernd nach den folgenden Gleichungen

$$P = \frac{1}{a} (PWG - b) \qquad (2)$$

$$P = \frac{1}{n} (m - PWLZ) \qquad (3)$$

berechnet werden, wobei a und b bzw. m und n patientenabhängige Konstanten sind, die in Vergleichsmessungen mit einem absoluten Blutdruckmeßgerät bestimmt werden können.

Zur Bestimmung der Pulswellenlaufzeit wird ein Elektrokardiogramm (EKG) aufgenommen und die auf die Herzkontraktionen zurückzuführenden Pulswellen werden an einer Körperstelle, die möglichst weit vom Herzen entfernt ist, beispielsweise an einem Finger des Patienten, detektiert. Wenn der Patient an eine extrakorporale Blutbehandlungseinrichtung angeschlossen ist, können die Pulswellen auch mit einem Drucksensor im extrakorporalen Kreislauf detektiert werden.

Figur 2a zeigt das Elektrokardiogramm, während Figur 2b das auf die Herzkontraktion zurückzuführende Pulswellensignal des Patienten zeigt. Als erster Bezugspunkt für die Messung der Pulswellenlaufzeit wird die R-Zacke im Elektrokardiogramm herangezo-

gen ($t_0$). Den zweiten Bezugspunkt ($t_1$) bildet der Druckanstieg der Pulswelle. Zur Bestimmung der Pulswellenlaufzeit wird die Zeitdauer zwischen den Zeitpunkten $t_0$ und $t_1$ bestimmt. In dem Pulswellensignal kann hierfür ein bestimmter Bezugspunkt C, beispielsweise an der ansteigenden Flanke des Pulswellensignals, festgelegt werden.

Aus der Pulswellenlaufzeit PWLZ = $t_1 - t_0$ kann dann der Blutdruck nach den obigen Gleichungen berechnet werden, wobei die patientenabhängigen Konstanten m und n durch Vergleichsmessungen mit einem konventionellen Blutdruckmeßgerät bestimmt werden.

Die Einrichtung 28 zur Überwachung der extrakorporalen Blutbehandlung weist zur Aufnahme des Elektrokardiogramms einen Elektrokardiographen 45 auf. Derartige Geräte sind bekannt, so daß sich eine weitere Erläuterung erübrigt. Desweiteren verfügt die Überwachungseinrichtung 28 über einen Photoplethysmographen 29 zur Detektion der Pulswellen am Finger des Patienten. Der Photoplethysmograph weist einen Klammer- oder Klebsensor 30 auf, der aus einer infraroten LED 31a und einer Photodiode 31b besteht. Der Elektrokardiograph 45 und die Einrichtung zur Detektion der Pulswellen sind über Datenleitungen 32, 33 mit einer Auswerteinheit 34 verbunden. Die Auswerteinheit 34 analysiert den Kurvenverlauf des Elektrokardiogramms und des Fingerpulssignals (Figur 2) und bestimmt die Pulswellenlaufzeit $t_1 - t_0$, die eine Angabe für den relativen Blutdruck darstellt. Hierzu umfaßt die Auswerteinheit eine Recheneinheit. In einem Komparator 36, der über eine Datenleitung 35 mit der Auswerteinheit 34 verbunden ist, wird die relative Änderung des Blutdrucks mit einem vorgegebenen Grenzwert verglichen. Wenn die relative Änderung des Blutdrucks unter den Grenzwert abfällt, erzeugt der Komparator ein Aktivierungs- bzw. Deaktivierungssignal, das die Steuereinheit 22 der Hämodialysevorrichtung über eine Datenleitung 37 empfängt.

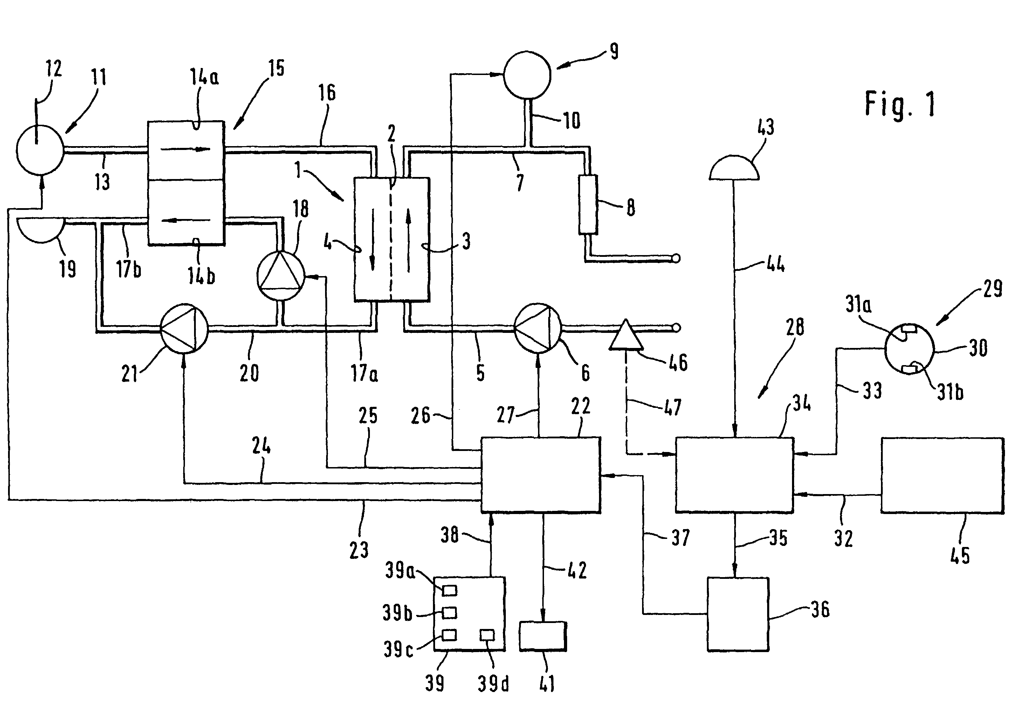

Die Bestimmung der Pulswellen kann entweder mit dem Photoplethysomographen 29 oder mit einem Drucksensor 46 erfolgen, der in den bekannten Hämodialysevorrichtungen zur arteriellen Drucküberwachung in der arteriellen Blutzuführleitung 5 stromauf der Blutpumpe 6 ohnehin vorgesehen ist. Die Auswerteinheit 34 empfängt das Pulssignal des Drucksensors 46 über eine Datenleitung 47. Da es sich bei der Detektion der Pulswellen mit dem Drucksensor 46 um eine alternative Ausführungsform handelt, ist die Datenleitung 47 in Figur 1 gestrichelt dargestellt. Prinzipiell ist es aber auch möglich, daß sowohl das Signal des Photoplethysomographen 29 als auch das Signal des Drucksensors 46 ausgewertet wird.

Die Steuereinheit 22 ist über eine Datenleitung 38 mit einer Eingabeeinheit 39 verbunden, über die der Benutzer vorgeben kann, welche Maßnahmen ergriffen werden sollen, um dem plötzlichen Blutdruckabfall entgegenzuwirken.

Ist der Taster 39a gedrückt, steuert die Steuereinheit 22 die Temperiereinheit 12 der Einrichtung 11 zur Aufbereitung der Dialysierflüssigkeit über die Steuerleitung 23 derart an, daß die Temperiereinheit eine niedrigere Temperatur für die Dialysierflüssigkeit einstellt, wenn die Steuereinheit 22 von dem Komparator 36 das Aktivierungs- bzw. Deaktivierungssignal empfängt, d.h. ein plötzlicher Blutdruckabfall auftritt. Ist der Schalter 39b gedrückt, steuert die Steuereinheit 22 bei einem plötzlichen Blutdruckabfall die Ultrafiltrationspumpe 21 über die Steuerleitung 24 derart an, daß diese angehalten wird. Die Einstellung einer niedrigeren Dialysierflüssigkeitstemperatur bzw. die Unterbrechung der Ultrafiltration kann für einen vorgegebenen Zeitraum erfolgen, es ist aber auch möglich, die Steuereinheit derart auszubilden, daß wieder zu dem normalen Behandlungsablauf dann übergegangen wird, wenn sich der Blutdruck wieder stabilisiert hat, d.h. die Steuereinheit 22 das Aktivierungs- bzw. Deaktivierungssignal nicht mehr empfängt. Hat der Benutzer den Schalter 39c gedrückt, aktiviert die Steuereinheit 22 bei einem plötzlichen Blutdruckabfall über die Steuerleitung 26 die Einrichtung 9 zur automatischen Applikation eines Bolus, beispielsweise 200 ml physiologische NaCl-Lösung, so daß sich der Blutdruck des Patienten wieder stabilisieren kann. Die Applikation eines Bolus kann einmalig oder für den Fall, daß sich der Blutdruck des Patienten nicht stabilisiert hat, mehrfach in unterschiedlichen Mengen erfolgen. Ist der Taster 39d gedrückt, steuert die Steuereinheit 22 die Dialysierflüssigkeitsaufbereitungseinrichtung 11 derart an, daß die Zusammensetzung der Dialysierflüssigkeit verändert wird.

Die Überwachungseinrichtung 28 weist ferner einen akustischen und/oder optischen Alarmgeber 41 auf, der bei einem plötzlichen Blutdruckabfall von der Steuereinheit 22 über eine Steuerleitung 42 aktiviert wird.

Die Steuereinheit 22 ist derart ausgebildet, daß die oben beschriebenen Maßnahmen zur Stabilisierung des Blutdrucks unabhängig voneinander oder in Kombination eingeleitet werden können.

Um auch absolute Werte für den systolischen und diastolischen bzw. mittleren Blutdruck messen und anzeigen zu können, weist die Hämodialysevorrichtung einen absoluten Blutdruckmesser 43 mit einer aufblasbaren Gummimanschette auf, die mit einem Manometer verbunden ist. Derartige Meßeinrichtungen zur indirekten Blutdruckmessung, die vollautomatisch arbeiten, sind bekannt, so daß sich eine nähere Beschreibung erübrigt. Das Ausgangssignal des Blutdruckmessers 43 wird über eine Datenleitung 44 der Auswerteinheit 34 zugeführt, die in der Recheneinheit aus den absoluten Meßwerten die patientenabhängigen Konstanten der Gleichung 3 bestimmt. Nach der Kalibrierung ist dann eine kontinuierliche Messung des Blutdrucks allein durch Bestimmung der Pulswellenlaufzeit möglich. Die gemessenen Blutdruckwerte können auch auf einer (nicht dargestellten Anzeigeeinheit) angezeigt werden. Die Manschettenmessung zur Bestimmung absoluter Blutdruckwerte kann dann automatisch gestartet werden, wenn die relativen Blutdruckwerte unter den vorgegebenen Grenzwert abfallen.

Figur 3 zeigt eine Hämofiltrationsvorrichtung mit einer Einrichtung zur kontinuierlichen, nicht invasiven Überwachung der extrakorporalen Blutbehandlung. Die Hämofiltrationsvorrichtung weist einen Hämofilter 1' auf, der durch eine semipermeable Membran 2' in eine erste Kammer 3' und eine zweite Kammer 4' getrennt ist. Der Einlaß der ersten Kammer 3' ist mit einem Ende einer Blutzuführleitung 5' verbunden, in die eine Blutpumpe 6' geschaltet ist, während der Auslaß der ersten Kammer 3' mit dem einen Ende einer Blutabführleitung 7' verbunden ist, in die eine Tropfkammer 8' geschaltet ist.

Die Hämofiltrationsvorrichtung verfügt über eine Einrichtung 11' zur Bereitstellung eines Substituats, die über eine Substituatleitung 16' , in die eine Kammer 14a' einer Bilanziereinrichtung 15' geschaltet ist, mit der Tropfkammer 8' in Verbindung steht. Von der zweiten Kammer 4' des Hämofilters 1' zweigt eine Filtratleitung 20' ab, die über die zweite Kammer 14b' der Bilanziereinrichtung 15' zu einem Auslaß 19' führt. In die Filtratleitung 20' ist stromauf der Bilanziereinrichtung 15' eine Ultrafiltrationspumpe 21' geschaltet.

Die Hämofiltrationsvorrichtung umfaßt ferner eine zentrale Steuereinheit 22, die über Steuerleitungen 27', 28' mit der Blutpumpe 6' und der Ultrafiltrationspumpe 21' verbunden ist.

Während der Hämofiltration wird dem Patienten mit der Ultrafiltrationspumpe 21' Flüssigkeit entzogen, während dem Patienten Substituat wieder zugefügt wird.

Die Überwachungseinrichtung 28' der Hämofiltrationsvorrichtung entspricht in ihrem Aufbau und ihrer Funktion der Überwachungseinrichtung der Hömodialysevorrichtung gemäß der Figuren 1 bis 3, so daß auf die obige Beschreibung Bezug genommen werden kann. Die Überwachungseinrichtung 28' weist einen Elektrokardiographen 45' zur Aufnahme des Elektrokardiograms und einen Photoplethysomographen 29' zur Detektion der Pulswellen am Finger der Patienten auf. Der Elektrokardiograph 45' und der Photoplethtysomograph 29' sind über Datenleitungen 32', 33' mit einer Auswerteinheit 34' verbunden, die den Kurvenverlauf des Elektrokardiograms und des Fingerpulssignals analysiert und die Pulswellenlaufzeit $t_1 - t_0$ bestimmt. In einem Komparator 36', der über eine Datenleitung 35' mit der Auswerteinheit 34' verbunden ist, wird die relative Änderung des Blutdrucks mit einem vorgegebenen Grenzwert verglichen. Wenn die relative Änderung des Blutdrucks unter den Grenzwert abfällt, erzeugt der Komparator 36' ein Aktivierungs - bzw. Deaktivierungssignal, das die Steuereinheit 22' der Hämofiltrationsvorrichtung über eine Datenleitung 37' empfängt.

Die Steuereinheit 22' ist über eine Datenleitung 38' mit einer Eingabeeinheit 39' verbunden, über die Benutzer wie bei der Hämodialysevorrichtung vorgeben kann, welche Maßnahmen ergriffen werden sollen, um dem plötzlichen Blutdruckabfall entgegenzuwirken.

Die Überwachungseinrichtung 28' weist ferner einen akustischen und/oder optischen Alarmgeber 41' auf, der bei einem plötzlichen Blutdruckabfall von der Steuereinheit 22' über eine Steuerleitung 42' aktiviert wird.

Ein absoluter Blutdruckmesser 43' mit einer aufblasbaren Gummimanschette, der über eine Datenleitung 44' mit der Auswerteinheit 34' verbunden ist, erlaubt die Bestimmung absoluter Blutdruckwerte.

Wie bei der unter Bezugnahme auf die Figuren 1 bis 3 beschriebenen Hämofiltrationsvorrichtung kann die Detektion der Pulswellen entweder mit dem Photoplethysomographen am Finger des Patienten oder erfindungsgemäß mit einem in der arteriellen Blutzuführleitung angeordneten Drucksensor 46' erfolgen.

## Patentansprüche

1. Vorrichtung zur extrakorporalen Blutbehandlung mit einer
   Blutzuführleitung (5, 5') eines extrakorporalen Blutkreislaufs zum Anschluss an einen Patienten oder Spender, die an einem Ende mit dem Einlass einer Blutbehandlungseinrichtung (1, 1') verbunden ist und an dem anderen Ende mit dem Gefäßsystem des Patienten oder Spenders verbindbar ist,
   Blutabführleitung (7, 7') des extrakorporalen Blutkreislaufs zum Anschluss an den Patienten oder Spender, die an einem Ende mit dem Auslass der Blutbehandlungseinrichtung (1, 1') verbunden ist und an dem anderen Ende mit dem Gefäßsystem des Patienten oder Spenders verbindbar ist,
   einer Einrichtung (28') zur kontinuierlichen Überwachung der extrakorporalen Blutbehandlungsvorrichtung, die aufweist:

   Mittel (34') zum Bestimmen einer mit dem Blutdruck korrelierenden Größe,

   Mittel (36') zum Vergleichen der mit dem Blutdruck korrelierenden Größe oder der relativen Änderung der mit dem Blutdruck korrelierenden Größe mit einem vorgegebenen Grenzwert und zur Erzeugung eines Aktivierungs- bzw. Deaktivierungssignals und

   eine das Aktivierungs- bzw. Deaktivierungssignal empfangende Steuereinheit (22') zur Einleitung eines Eingriffs in den Behandlungsablauf, wenn das Aktivierungs- bzw. Deaktivierungssignal erzeugt wird,

   **dadurch gekennzeichnet,**
   **dass** die Mittel (34') zum Bestimmen einer mit dem Blutdruck korrelierenden Größe derart ausgebildet sind, dass die mit dem Blutdruck korrelierende Größe aus der Ausbreitungsgeschwindigkeit der Pulswellen oder Pulswellenlaufzeit bestimmbar ist, und **dass** die Einrichtung (28') zur kontinuierlichen Überwachung der extrakorporalen Blutbehandlungsvorrichtung ferner Mittel (45', 29', 34', 36') zum Bestimmen der Ausbreitungsgeschwindigkeit der sich über das arterielle Gefäßsystem des Patienten fortpflanzenden Pulswellen, die durch dessen Herzkontraktionen erzeugt werden, oder der Laufzeit der Pulswellen über eine Strecke des Gefäßsystems mit einer vorgegebenen Länge aufweisen, wobei die Mittel zum Bestimmen der Ausbreitungsgeschwindigkeit oder Laufzeit aufweisen:

eine Einrichtung zur Detektion der Pulswellen, die ein im extrakorporalen Kreislauf angeordneter Drucksensor (29, 29') ist, eine Einrichtung (45) zur Ermittlung eines Elektrokardiogramms und eine Einrichtung (34) zum Bestimmen der Zeit zwischen einem Bezugszeitpunkt to, zu dem eine auf die Herzkontraktion zurückzuführende Pulswelle zu laufen beginnt, und dem Zeitpunkt $t_1$, zu dem die Pulswelle detektiert wird.

2. Vorrichtung nach Anspruch 1, **dadurch gekennzeichnet, dass** der Drucksensor im extrakorporalen Kreislauf stromauf der Blutbehandlungseinrichtung (1, 1') angeordnet ist.

3. Blutbehandlungsvorrichtung nach Anspruch 1 oder 2, **dadurch gekennzeichnet, dass** die Einrichtung (34) eine Einrichtung zum Bestimmen der Zeit zwischen dem Zeitpunkt to ist, zu dem die R-Zacke im Elektrokardiogramm auftritt, und dem Zeitpunkt $t_1$, zu dem die Pulswelle detektiert wird.

4. Blutbehandlungsvorrichtung nach einem der Ansprüche 1 bis 3, **dadurch gekennzeichnet, dass** die Mittel (34) zum Bestimmen einer mit dem Blutdruck korrelierenden Größe eine Recheneinheit aufweisen, die derart ausgebildet ist, dass aus der Pulswellenlaufzeit der Blutdruck nach der folgenden Gleichung bestimmbar ist,

$$P = \frac{1}{n} \, (m - PWLZ)$$

wobei m, n patientenabhängige Konstanten sind.

5. Blutbehandlungsvorrichtung nach einem der Ansprüche 1 bis 4, **dadurch gekennzeichnet, dass** eine Einrichtung (9) zur Applikation eines Bolus, insbesondere einer vorbestimmten Menge einer NaCl-Lösung, in die Blutzuführ- oder -abführleitung vorgesehen ist, die von der Steuereinheit (22) dann aktiviert wird, wenn das Aktivierungs- bzw. Deaktivierungssignal erzeugt wird.

6. Blutbehandlungsvorrichtung nach einem der Ansprüche 1 bis 5, **dadurch gekennzeichnet, dass** ein akustischer oder optischer Alarmgeber (41) vorgesehen ist, der von der Steuereinheit (22) dann aktiviert wird, wenn das Aktivierungs- bzw. Deaktivierungssignal erzeugt wird.

7. Vorrichtung nach einem der Ansprüche 1 bis 6, **dadurch gekennzeichnet, dass** die Blutbehandlungseinrichtung einen Hämofilter (1') umfasst, der eine erste Kammer (3') und eine zweite Kammer (4') aufweist, die von einer semipermeablen Membran (2') getrennt sind, wobei die Blutzuführleitung (5') mit dem Einlass der ersten Kammer (3') und die Blutabführleitung (7') mit dem Auslass der ersten Kammer verbunden und in Blutzuführ- oder abführleitung (5', 7') eine Blutpumpe (6') geschaltet ist, und dass eine von der zweiten Kammer (4') des Hämofilters (1') abzweigende und zu einem Auslass führende Filtratleitung vorgesehen ist, in die eine Ultrafiltrationsleitung geschaltet ist.

8. Vorrichtung nach einem der Ansprüche 1 bis 6, **dadurch gekennzeichnet, dass** die Blutbehandlungseinrichtung einen Dialysator (1) umfasst, der eine Blutkammer (3) und eine Dialysierflüssigkeitskammer (4) aufweist, die von einer semipermeablen Membran (2) getrennt sind, wobei die Blutzuführleitung (5) mit einem Einlass der Blutkammer (3) und die Blutabführleitung (7) mit einem Auslass der Blutkammer verbunden ist, und dass eine Dialysierflüssigkeitsquelle (11), die über eine Dialysierflüssigkeitszuhrleitung (13, 16) mit einem Einlass der Dialysierflüssigkeitskammer verbunden ist, eine mit einem Auslass der Dialysierflüssigkeitskammer verbundene Dialysierflüssigkeitsabführleitung (17a, 17b), eine in die Blutzuführ- oder abführleitung geschaltete Blutpumpe (6) und einen in die Dialysierflüssigkeitszuführ- oder abführleitung geschaltete Dialysierflüssigkeitspumpe (18) vorgesehen sind

9. Blutbehandlungsvorrichtung nach Anspruch 8, **dadurch gekennzeichnet, dass** eine mindestens zwei Bilanzkammerhälften (14a, 14b) aufweisende Bilanziereinrichtung (15) vorgesehen ist, deren eine Bilanzkammerhälfte (14a) in die Dialysierflüssigkeitzuführleitung (13, 16) und deren andere Bilanzkanunerhälfte (14b) in die Dialysierflüssigkeitsabführleitung (17 a, 17b) geschaltet ist, und eine Ultrafiltrationseinrichtung vorgesehen ist, die eine von der Dialysierflüssigkeitsabführleitung stromauf der Bilanziereinrichtung abzweigende Ultrafiltratabführleitung (20) aufweist, in die eine Ultrafiltrationspumpe (21) geschaltet ist, die von der Steuereinheit (22) dann deaktiviert wird, wenn das Aktivierungs- bzw. Deaktivierungssignal erzeugt wird.

10. Blutbehandlungsvorrichtung nach Anspruch 8 oder 9, **dadurch gekennzeichnet, dass** die Dialysierflüssigkeitsquelle (11) eine Temperiereinheit (12) aufweist, die derart ausgebildet ist, dass mindestens zwei unterschiedliche Temperaturwerte einstellbar sind, wobei die Temperiereinheit von der Steuereinheit (22) auf die niedrigere Temperatur einstellbar ist, wenn das Aktivierungs- bzw. Deaktivierungssignal erzeugt wird.

11. Blutbehandlungsvorrichtung nach einem der Ansprüche 8 bis 10, **dadurch gekennzeichnet, dass** eine Einrichtung (11') zur Aufbereitung der Dialysierflüssigkeit vorgesehen ist, die derart ausgebildet ist, dass mindestens zwei unterschiedliche Dialysierflüssigkeitszusammensetzungen einstellbar sind, wobei die Einrichtung zur Aufbereitung der Dialysierflüssigkeit von der Steuereinheit (22') auf eine der mindestens zwei Dialysierflüssigkeitszusammensetzungen einstellbar ist, wenn das Aktivierungs- bzw. Deaktivierungssignal erzeugt wird.

**Claims**

1. A device for extracorporeal blood treatment with a blood supply line (5, 5') of an extracorporeal blood circuit for connection to a patient or donor, said blood supply line being connected at one end to the inlet of a blood treatment device (1, 1') and being connectable at the other end to the vessel system of the patient or donor,
blood supply line (7, 7') of an extracorporeal blood circuit for connection to the patient or donor, said blood supply line being connected at one end to the outlet of a blood treatment device (1, 1') and being connectable at the other end to the vessel system of the patient or donor,
a device (28') for the continuous monitoring of the extracorporeal blood treatment device, which has:

   means (34') for determining a magnitude correlating with the blood pressure,

   means (36') for comparing the magnitude correlating with the blood pressure or the relative change in the magnitude correlating with the blood pressure with a preset limiting value and for generating an activation or deactivation signal and

   a control unit (22') receiving the activation or deactivation signal for initiating an intervention in the treatment sequence when the activation or deactivation signal is generated,

**characterised in that**
the means (34') for determining a magnitude correlating with the blood pressure are designed in such a way that the magnitude correlating with the blood pressure can be determined from the propagation rate of the pulse waves or pulse-wave running time, and
that the device (28') for the continuous monitoring of the extracorporeal blood treatment device also has means (45', 29', 34', 36') for determining the propagation rate of the pulse waves being propagated via the arterial vessel system of the patient, said pulse waves being generated by heart contractions of said patient, or the running time of the pulse waves over a section of the vessel system with a predetermined length,
whereby the means for determining the propagation rate or the running time have:

   a device for detecting the pulse waves, which is a pressure sensor (29, 29') arranged in the extracorporeal circuit, a device (45) for determining an electrocardiogram and a device (34) for determining the time between a reference time-point $t_0$, at which a pulse wave traceable back to the heart contraction begins to propagate, and the time-point $t_1$ at which the pulse wave is detected.

2. The device according to claim 1, **characterised in that** the pressure sensor is arranged in the extracorporeal circuit upstream of the blood treatment device (1, 1').

3. The blood treatment device according to claim 1 or 2, **characterised in that** the device (34) is a device for determining the time between the time-point $t_0$, at which the R-spike occurs in the electrocardiogram, and the time-point $t_1$ at which the pulse wave is detected.

4. The blood treatment device according to any one of claims 1 to 3, **characterised in that** the means (34) for determining a magnitude correlating with the blood pressure is a computing unit which is designed in such a way that the blood pressure can be determined from the pulse-wave running time according to the following equation,

$$P = 1/n \ (m - PWLZ)$$

whereby m, n are patient-dependent constants.

5. The blood treatment device according to any one of claims 1 to 4, **characterised in that** a device (9) is provided for the application of a bolus, in particular a predetermined quantity of NaCl solution, into the blood supply or discharge line, said device being activated by the control unit (22) when the activation or deactivation signal is generated.

6. The blood treatment device according to any one of claims I to 5, **characterised in that** an acoustic or optical alarm transmitter (41) is provided, which is activated by the control unit (22) when the activation or deactivation signal is generated.

7. The device according to any one of claims 1 to 6, **characterised in that** the blood treatment device includes a haemofilter (1'), which has a first chamber (3') and a second chamber (4'), which are separated by a semipermeable membrane (2'), whereby the blood supply line (5') is connected to the inlet of the first chamber (3') and the blood discharge line (7') is connected to the outlet of the first chamber and a blood pump (6') is connected into (7') blood supply or discharge line (5', 7'), and that a filtrate line branching off from the second chamber (4') of the haemofilter (1') and leading to an outlet is provided, into which filtrate line an ultrafiltration line is connected.

8. The device according to any one of claims 1 to 6, **characterised in that** the blood treatment device includes a dialyser (1), which has a blood chamber (3) and a dialysis-liquid chamber (4) which are separated by a semipermeable membrane (2), whereby the blood supply line (5) is connected to an inlet of the blood chamber (3) and the blood discharge line (7) is connected to an outlet of the blood chamber, and that a dialysis-liquid source (11) connected via a dialysis-liquid supply line (13, 16) to an inlet of the dialysis-liquid chamber, a dialysis-liquid discharge line (17a, 17b) connected to an outlet ofthe dialysis-liquid chamber, a blood pump (6) connected into the blood supply or discharge line and a dialysis-liquid pump (18) connected into the dialysis-liquid supply or discharge line are provided.

9. The blood treatment device according to claim 8, **characterised in that** a balancing device (15) having at least two balancing-chamber halves (14a, 14b) is provided, one balancing-chamber half (14a) of which balancing device is connected into the dialysis-liquid supply line (13, 16) and the other balancing-chamber half (14b) of which balancing device is connected into the dialysis-liquid discharge line (17a, 17b), and an ultrafiltration device is provided which has an ultra-filtrate discharge line (20) branching off from the dialysis-liquid discharge line upstream of the balancing device, into which ultra-filtrate discharge line an ultrafiltration pump (21) is connected, which is deactivated by the control unit (22) when the activation or deactivation signal is generated.

10. The blood treatment device according to claim 8 or 9, **characterised in that** the dialysis-liquid source (11) has a temperature-regulating unit (12), which is designed in such a way that at least two different temperature values can be set, whereby the temperature-regulating unit can be set by the control unit (22) to the lower temperature when the activation or deactivation signal is generated.

11. The blood treatment device according to any one of claims 8 to 10, **characterised in that** a device (11') for the preparation of the dialysis liquid is provided, said device being designed in such a way that at least two different dialysis-liquid compositions can be set, whereby the device for the preparation of the dialysis liquid can be set by the control unit (22') to one of the at least two dialysis-liquid compositions when the activation or deactivation signal is generated.

## Revendications

1. Dispositif pour le traitement extracorporel du sang avec

un conduit d'amenée de sang (5, 5') d'un circuit de sang extracorporel pour le raccordement à un patient ou donneur, qui est relié à une extrémité avec l'entrée d'un dispositif de traitement du sang (1, 1') et qui peut être relié à l'autre extrémité avec le système vasculaire du patient ou donneur,

un conduit d'évacuation de sang (7, 7') du circuit de sang extracorporel pour le raccordement au patient ou donneur, qui est relié à une extrémité avec la sortie du dispositif de traitement du sang (1, 1') et qui peut être relié à l'autre extrémité avec le système vasculaire du patient ou donneur,

un dispositif (28') pour la surveillance continue du dispositif de traitement extracorporel du sang, qui comprend :

des moyens (34') pour déterminer une grandeur corrélée avec la pression sanguine,
des moyens (36') pour comparer la grandeur corrélée avec la pression sanguine ou la modification relative de la grandeur corrélée avec la pression sanguine avec une valeur limite prédéterminée et pour produire un signal d'activation ou de désactivation et
une unité de commande (22') recevant le signal d'activation ou de désactivation pour engager une intervention dans le déroulement du traitement quand le signal d'activation ou de désactivation est produit, **caractérisé**
**en ce que** les moyens (34') pour déterminer une grandeur corrélée avec la pression sanguine sont agencés de telle manière que la grandeur corrélée avec la pression sanguine peut être déterminée d'après la vitesse de diffusion des ondes d'impulsion ou d'après la durée de parcours des ondes d'impulsion, et **en ce que** le dispositif (28') pour la surveillance continue du dispositif de traitement extracorporel du sang comporte en outre des moyens (45', 29', 34', 36') pour déterminer la vitesse de diffusion des ondes d'impulsion qui se propagent dans le système vasculaire artériel du patient, qui sont produites par ses contractions cardiaques, ou la durée de parcours des ondes d'impulsion sur une section du système vasculaire d'une longueur prédéterminée,

où les moyens pour déterminer la vitesse de diffusion ou la durée de parcours comportent :

un dispositif pour détecter les ondes d'impulsion qui est un capteur de pression (29, 29') disposé dans le circuit extracorporel, un dispositif (45) pour déterminer un électrocardiogramme et un dispositif (34) pour déterminer la durée entre un instant de référence $t_0$ auquel une onde d'impulsion à attribuer à la contraction cardiaque commence à se déplacer et l'instant $t_1$ auquel l'onde d'impulsion est détectée.

2. Dispositif selon la revendication 1 **caractérisé en ce que** le capteur de pression est disposé dans le circuit extracorporel en amont du dispositif de traitement du sang (1, 1').

3. Dispositif de traitement du sang selon la revendication 1 ou 2 **caractérisé en ce que** le dispositif (34) est un dispositif pour déterminer la durée entre l'instant $t_0$ auquel la pointe R apparaît dans l'électrocardiogramme et l'instant $t_1$ auquel l'onde d'impulsion est détectée.

4. Dispositif de traitement du sang selon l'une des revendications 1 à 3 **caractérisé en ce que** les moyens (34) pour déterminer une grandeur corrélée avec la pression sanguine comportent une unité de calcul qui est agencée de telle manière que la pression sanguine peut être déterminée à partir de la durée de parcours des ondes d'impulsion d'après l'équation suivante :

$$P = \frac{1}{n}(m - DPOI)$$

où m, n sont des constantes dépendantes du patient.

5. Dispositif de traitement du sang selon l'une des revendications 1 à 4 **caractérisé en ce qu'**il est prévu dans le conduit d'amenée ou d'évacuation du sang un dispositif (9) pour l'application d'un bol, en particulier d'une quantité prédéterminée d'une solution de NaCl, qui est activé par l'unité de commande (22) quand le signal d'activation ou de désactivation est produit.

6. Dispositif de traitement du sang selon l'une des revendications 1 à 5 **caractérisé en ce qu'**il est prévu un dispositif d'alarme acoustique ou optique (41) qui est activé par l'unité de commande (22) quand le signal d'activation ou de désactivation est produit.

7. Dispositif selon l'une des revendications 1 à 6 **caractérisé en ce que** le dispositif de traitement du sang comprend un hémofiltre (1') qui comporte une première chambre (3') et une deuxième chambre (4') qui sont séparées par une membrane semi-perméable (2'), où le conduit d'amenée de sang (5') est relié avec l'entrée de la première chambre (3') et le conduit d'évacuation de sang (7') est relié avec la sortie de la première chambre et une pompe à sang (6') est branchée dans le conduit d'amenée ou d'évacuation de sang (5', 7'), et **en ce qu'**il est prévu un conduit de filtrat dérivant de la deuxième chambre (4') de l'hémofiltre (1') et menant à une sortie, dans lequel est branché un conduit d'ultra-filtration.

8. Dispositif selon l'une des revendications 1 à 6 **caractérisé en ce que** le dispositif de traitement du sang comprend un dialyseur (1) qui comprend une chambre à sang (3) et une chambre à liquide de dialyse (4) qui sont séparées par une membrane semi-perméable (2), où le conduit d'amenée de sang (5) est relié avec une entrée de la chambre à sang (3) et le conduit d'évacuation de sang (7) est relié avec une sortie de la chambre à sang, et **en ce qu'**il est prévu une source de liquide de dialyse (11) qui est reliée par le biais d'un conduit d'amenée de liquide de dialyse (13, 16) avec une entrée de la chambre à liquide de dialyse, un conduit d'évacuation de liquide de dialyse (17a, 17b) relié avec une sortie de la chambre à liquide de dialyse, une pompe à sang (6) branchée dans le conduit d'amenée ou d'évacuation de sang et une pompe à liquide de dialyse (18) branchée dans le conduit d'amenée ou d'évacuation de liquide de dialyse.

9. Dispositif de traitement du sang selon la revendication 8 **caractérisé en ce qu'**il est prévu un dispositif d'établissement de bilan (15) comportant au moins deux moitiés de chambre de bilan (14a, 14b) dont une moitié de chambre de bilan (14a) est branchée dans le conduit d'amenée de liquide de dialyse (13, 16) et dont l'autre moitié de chambre de bilan (14b) est branchée dans le conduit d'évacuation de liquide de dialyse (17a, 17b), et il est prévu un dispositif d'ultrafiltration qui comprend un conduit d'évacuation d'ultrafiltrat (20) dérivant du conduit d'évacuation de liquide de dialyse en amont du dispositif d'établissement de bilan, dans lequel est branchée une pompe d'ultrafiltration (21) qui est désactivée par l'unité de commande (22) quand le signal d'activation ou de désactivation est produit.

**10.** Dispositif de traitement du sang selon la revendication 8 ou 9 **caractérisé en ce que** la source de liquide de dialyse (11) comprend une unité thermostatique (12) qui est agencée de telle manière qu'il est possible de régler au moins deux valeurs de température différentes, où l'unité thermostatique peut être réglée par l'unité de commande (22) à la température la plus basse quand le signal d'activation ou de désactivation est produit.

**11.** Dispositif de traitement du sang selon l'une des revendications 8 à 10 **caractérisé en ce qu'**il est prévu un dispositif (11') pour la préparation du liquide de dialyse qui est agencé de telle manière qu'il est possible de régler au moins deux compositions de liquide de dialyse différentes, où le dispositif pour la préparation du liquide de dialyse peut être réglé par l'unité de commande (22') sur l'une des deux ou plus de deux compositions de liquide de dialyse, quand le signal d'activation ou de désactivation est produit.

Fig. 1

EP 0 911 044 B1

Fig. 2a

Fig. 2b

Fig. 3

EP 0 911 044 B1